# EUROPEAN PATENT APPLICATION

(11) **EP 4 431 600 A1**
(43) Date of publication of application: **18.09.2024**
(21) Application number: 22892880.0
(22) Date of filing: 10.11.2022
(51) Int. Cl.: C12N 7/02, B01D 15/08, B01D 61/16, C12N 15/864

(54) **VIRUS PURIFICATION METHOD**

(30) Priority: 12.11.2021 JP 2021184941
(71) Applicant: AGC INC., Chiyoda-ku, Tokyo 1008405 (JP)
(72) Inventor: OHTAKI, Kaya, Tokyo 100-8405 (JP); OKAMOTO, Mayumi, Tokyo 100-8405 (JP); SHIINA, Shunsuke, Tokyo 100-8405 (JP)
(74) Representative: Müller-Boré & Partner Patentanwälte PartG mbB
(86) International application number: PCT/JP2022/041990
(87) International publication number: WO 2023/085382

(57) **Abstract**

The present invention aims to provide methods for purifying viruses, including an ultrafiltration step, which can purify the virus more conveniently with a high purity. Virus can be obtained in a high yield by ultrafiltration of an aqueous dispersion containing the virus, a surfactant, and an inorganic salt.

## Description

### [Technical Field]

The present invention relates to purification methods of viruses, particularly methods for purifying viruses, including an ultrafiltration step.

### [Background Art]

In medical fields such as gene therapy and the like, methods using virus-derived vectors for gene transfer (hereinafter viral vector) are generally employed as methods for introducing gene into cells of mammals inclusive of human. Viral vector refers to a recombinant virus obtained by altering a naturally occurring virus by gene recombination technology so that a desired gene and the like can be transferred into the target, and the technical development is ongoing in recent years.

As viruses from which viral vectors are derived, viruses with envelopes such as retrovirus, lentivirus, sendai virus, herpes virus, and the like, and viruses without envelopes (hereinafter referred to as non-enveloped viruses) such as adenovirus, adeno-associated virus (hereinafter AAV), and the like are well known.

Particularly, AAV is seen as a promising vector for gene transfer used in gene therapy methods because it can infect a wide range of cell types including humans, can also infect non-dividing cells that have completed differentiation, is little concerned about side effects by being not pathogenic to humans, and has physicochemically stable virus particles.

In order to utilize recombinant viruses for gene therapy and the like, it is necessary to obtain purified viruses, preferably in large amounts. For example, Non Patent Literature 1 discloses a technique for producing recombinant adeno-associated virus for clinical tests. As a method for obtaining purified viruses, there is a method for purifying viruses in which cells that produce viruses are produced by transiently or permanently introducing nucleic acids that supply elements essential for virus particle formation into cells, after which the virus-producing cells are collected and disrupted to obtain a cell lysate (aqueous dispersion) containing the virus, and the obtained aqueous dispersion is subjected to appropriate steps such as filter filtration, ultracentrifugation, chromatography, and ultrafiltration. For example, it is known that viruses can be separated from water, ionic components, low-molecular-weight compounds, host cell-derived proteins, and the like in the culture solution by using an ultrafiltration membrane (UF membrane).

Purification and separation of viruses by using ultracentrifugation is a conventional method but is not suitable for scale-up. Non Patent Literature 2 discloses a virus purification protocol that does not require ultracentrifugation. In the protocol, Tangential Flow Filtration (TFF) technique is used, but the yield is not satisfactory.

Cell lysate and the like contain impurities such as protein and the like, and separation of virus therefrom, particularly, purification of the virus to a high purity, requires complicated steps and poses a problem in terms of cost.

### [Citation List]

### [Non Patent Literature]

[Non Patent Literature 1]
   Clement, Nathalie et al, Mol Ther Methods Clin Dev. 2016 Mar 16; 3:16002.
[Non Patent Literature 2]
   Tomono, Taro et al., Mol Ther Methods Clin Dev. 2018 Nov 1; 11:180-190.

### [Summary of Invention]

### [Technical Problem]

The problem of the resent invention is to provide purification methods of viruses, particularly, purification methods of viruses including an ultrafiltration step that enable more convenient virus purification and afford viruses in high yields.

### [Solution to Problem]

In view of the above-mentioned problem, the present inventors have intensively studied various conditions during the ultrafiltration step in virus purification. As a result, they have found that the yield of virus purification can be increased by adjusting the surfactant concentration, salt concentration, and temperature during the ultrafiltration step, and succeeded in determining the preferred concentration range, which resulted in the completion of the present invention.

Accordingly, the present invention provides the following.

[1] A method for purifying a virus, comprising a step of ultrafiltrating an aqueous dispersion comprising a virus, a surfactant, and an inorganic salt.
[2] The purification method of [1], wherein the aforementioned virus is derived from a virus belonging to Parvoviridae or Retroviridae.
[3] The purification method of [1] or [2], wherein the aforementioned inorganic salt is an inorganic salt containing at least one selected from the group consisting of chloride ion, sulfide ion, phosphate ion, and carbonate ion.
[4] The purification method of any of [1] to [3], wherein the aforementioned inorganic salt is at least one selected from the group consisting of sodium chloride, magnesium chloride, and magnesium sulfate.
[5] The purification method of any of [1] to [4], wherein the aforementioned surfactant is at least one selected from the group consisting of a non-ionic surfactant, an anionic surfactant, a cationic surfactant, and a zwitterionic surfactant.
[6] The purification method of any of [1] to [5], wherein a content of the aforementioned surfactant is 0.01 to 2.0 mass % with respect to the total amount of the aforementioned aqueous dispersion.
[7] The purification method of any of [1] to [6], wherein a content of the aforementioned inorganic salt is 80 to 800 mM with respect to the total amount of the aforementioned aqueous dispersion.
[8] The purification method of any of [1] to [7], further comprising a step of extracting a virus from a cell containing the virus and obtaining the aforementioned aqueous dispersion, which step is performed before the ultrafiltration step.
[9] The purification method of any of [1] to [8], further comprising a step of purifying by column chromatography, which step is performed after the ultrafiltration step.
[10] The purification method of [9], wherein the aforementioned column chromatography is at least one selected from the group consisting of affinity chromatography, ion exchange chromatography, hydrophobic chromatography, gel filtration chromatography, and any combination of these.
[11] A method for purifying a virus, comprising
   (a) a step of extracting a virus from a cell containing the virus and obtaining an aqueous dispersion containing the virus, a surfactant, and an inorganic salt,
   (b) a step of performing ultrafiltration of the aforementioned aqueous dispersion to obtain a crude liquid containing the virus, and
   (c) a step of purifying the aforementioned crude liquid by column chromatography.
[12] A method for purifying a virus, comprising a step of performing ultrafiltration of an aqueous dispersion containing the virus, a surfactant, and an inorganic salt, wherein
   the aforementioned virus is an adeno-associated virus,
   the serotype of the aforementioned adeno-associated virus is at least one selected from the group consisting of AAV5 and AAV6, and
   the temperature of the aforementioned ultrafiltration is 28 to 50°C.
[13] The purification method of [12], wherein the aforementioned inorganic salt is an inorganic salt containing at least one selected from the group consisting of chloride ion, sulfide ion, phosphate ion, and carbonate ion.
[14] The purification method of [12] or [13], wherein the aforementioned inorganic salt is at least one selected from the group consisting of sodium chloride, magnesium chloride, and magnesium sulfate.
[15] The purification method of any of [12] to [14], wherein the aforementioned surfactant is at least one selected from the group consisting of a non-ionic surfactant, an anionic surfactant, a cationic surfactant, and a zwitterionic surfactant.
[16] The purification method of any of [12] to [15], wherein a content of the aforementioned surfactant is 0.01 to 2.0 mass % with respect to the total amount of the aforementioned aqueous dispersion.
[17] The purification method of any of [12] to [16], wherein a content of the aforementioned inorganic salt is 80 to 800 mM with respect to the total amount of the aforementioned aqueous dispersion.
[18] The purification method of any of [12] to [17], further comprising a step of extracting a virus from a cell containing the virus and obtaining the aforementioned aqueous dispersion, which step is performed before the ultrafiltration step.
[19] The purification method of any of [12] to [18], further comprising a step of purifying by column chromatography, which step is performed after the ultrafiltration step.
[20] The purification method of [19], wherein the aforementioned column chromatography is at least one selected from the group consisting of affinity chromatography, ion exchange chromatography, hydrophobic chromatography, gel filtration chromatography, and any combination of these.

### [Advantageous Effects of Invention]

According to the method of the present invention, it becomes possible to purify viruses more conveniently at a low cost in a high yield.

### [Brief Description of Drawings]

[Fig. 1]

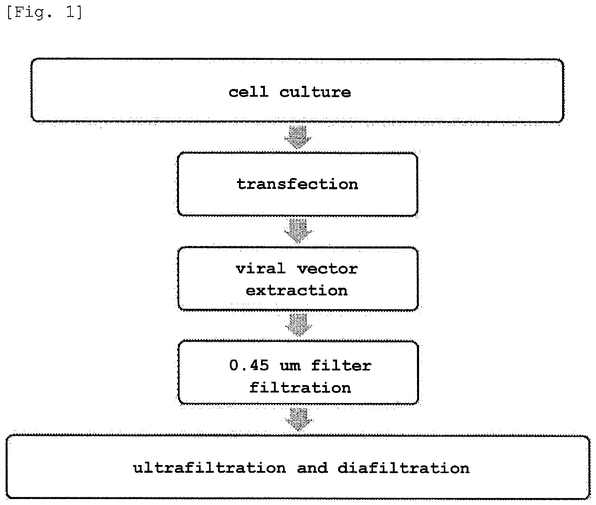

Fig. 1 is a chart showing the procedure for virus purification.

### [Description of Embodiments]

The present invention is explained below. The terms used in the present specification have meanings generally used in the pertinent field, unless particularly specified.

The present invention provides purification methods of viruses, and the method is characterized by including a step of performing ultrafiltration of an aqueous dispersion containing a virus, a surfactant, and an inorganic salt.

### 1. Virus

Examples of the virus to be the target of purification in the present invention include, but are not limited to, viruses with envelopes such as retrovirus and lentivirus, sendai virus, herpes virus, and the like, viruses without envelope (hereinafter non-enveloped viruses) such as adenovirus, AAV and the like, and the like. The envelope is formed when the virus buds through membranes of nucleus, endoplasmic reticulum, Golgi apparatus, plasma membrane, cell membrane, and the like, generally accompanied by host-derived proteins or viral proteins expressed on the cell membrane of the host, and plays an important role in infecting target cells.

Specifically, DNA viruses such as adenovirus, parvovirus, papovavirus, human papillomavirus, and the like, and RNA viruses such as rotavirus, coxsackievirus, enterovirus, sapovirus, norovirus, poliovirus, echovirus, hepatitis A virus, hepatitis E virus, rhinovirus, astrovirus, and the like can be mentioned. Retroviridae virus, Adenoviridae virus, and Parvoviridae virus are more preferred, and adeno-associated virus (AAV) of Parvoviridae is particularly preferred. AAV has a regular icosahedral outer shell (capsid) without an envelope and one linear single-stranded DNA in the inside thereof. The capsid has three capsid proteins (VP1, VP2, and VP3). In the present specification, AAV includes wild-type viruses and derivatives thereof, and includes all serotypes and clades unless particularly specified. There are various reports regarding the serotypes of AAV, but at least 15 types of AAV1, AAV2, AAV3a, AAV3b, AAV4, AAV5, AAV6, AAV7, AAV8, AAV9, AAV10, AAVrh.10, AAV11, AAV12, and AAV13 are known as the serotypes of AAV that infect humans.

In the present invention, the "virus" includes not only natural viruses but also recombinant virus particles that have been modified based on natural viruses to remove pathogenicity and include a region for introducing foreign genes. The "virus particle" includes not only particles containing a viral genome (in the form of nucleic acid) but also hollow particles that are virus-like particles that do not contain a viral genome. Such recombinant virus particles are also to be referred to as viral vectors. In the present invention, natural viruses and recombinant virus particles (viral vectors) are collectively referred to as viruses.

Therefore, the purification method of virus of the present invention can also be a purification method of recombinant virus particles or a purification method of viral vectors.

### 2. Surfactant

The surfactant used in the present invention is not particularly limited as long as it enables ultrafiltration to be performed with high yield. It may be any as long as it permits, in addition to extracting viruses from cells containing viruses (described later), suppressing aggregation of proteins derived from virus-containing cells and adsorption of viruses to membranes during ultrafiltration, and may be selected as appropriate depending on the cells to be the target and the like. Generally, it is at least one selected from the group consisting of non-ionic surfactant, anionic surfactant, cationic surfactant, and zwitterionic surfactant, and preferably one selected from the group. More preferably, it is a non-ionic surfactant or a zwitterionic surfactant.

Non-ionic surfactants refer to those that do not form ion in water. As the non-ionic surfactant, known non-ionic surfactants can be used. They are classified into ester type, ether type, ester ether type, alkanolamide type, sugar type, and methylglucamine type. Examples of the aforementioned ester type include polyoxyethylene sorbitan monolaurate (e.g., Tween (registered trademark) 20), sorbitan polyoxyethylene monopalmitate (e.g., Tween (registered trademark) 40), polyoxyethylene sorbitan monostearate (e.g., Tween (registered trademark) 60), polyoxyethylene sorbitan monooleate (e.g., Tween (registered trademark) 80), and the like. Examples of the ether type include polyoxyethylene alkyl ether (e.g., BriJ (registered trademark) L23, BriJ (registered trademark) L58), and polyoxyethylene isooctyl phenyl ether (e.g., Triton-X 100 (registered trademark)). Examples of the ester ether type include polyoxyethylene sorbitan fatty acid ester, polyoxyethylenehexitan fatty acid ester, sorbitan fatty acid ester polyethylene glycol, and polyoxyethylene-polyoxypropylene block copolymer (e.g., Pluronic (registered trademark)). Furthermore, examples of the alkanolamide include N,N-bis[3-(D-gluconamido)propyl]deoxycholamide. Examples of the sugar type include alkylmaltoside and alkylglucopyranoside. Examples of the methylglucamine type include alkyl-N-methylglucamine.

Anionic surfactants refer to those that ionize in water and become organic anions. As the anionic surfactant, known anionic surfactants can be used. They are classified into carboxylic acid type, sulfonic acid type, sulfate ester type, and phosphate ester type. Examples of the carboxylic acid type include deoxycholate, cholate, and lauroylsarcosinate. Examples of the sulfonic acid type include dodecyl sulfate. The aforementioned salt is preferably a sodium salt or a lithium salt.

Cationic surfactants refer to those that ionize in water and become organic cations. As the cationic surfactant, known cationic surfactants can be used. They are classified into alkylamine salt type and quaternary ammonium salt type. Examples of the alkylamine salt type include monomethylamine hydrochloride, dimethylamine hydrochloride, and trimethylamine hydrochloride. Examples of the quaternary ammonium salt type include hexadecyltrimethylammonium bromide, myristyltrimethylammonium bromide, and the like.

Zwitterionic surfactants refer to surfactants that have both anionic and cationic groups in a molecule. As the zwitterionic surfactant, known zwitterionic surfactants can be used. Examples thereof include alkylamino fatty acid salt, sulfobetaine, alkylbetaine, alkylaminoxide, and the like. It is preferably alkylbetaine. Examples of the alkylbetaine include 3-[(3-cholamidopropyl)dimethyl-ammonio]propanesulfonate (CHAPS).

### 3. Inorganic salt

The type of the inorganic salt of the present invention is not particularly limited as long as it can be used to achieve the desired purposes, that is, the effect of suppressing virus aggregation and adsorption to an ultrafiltration membrane, and the effect of suppressing adsorption to an ultrafiltration membrane due to non-specific binding of viruses. Generally, it is an inorganic salt containing at least one kind selected from the group consisting of phosphate ion, sulfide ion, acetate ion, chloride ion, bromide ion, nitrate ion, carbonate ion, chlorate ion, iodide ion, thiocyanate ion, ammonium ion, rubidium ion, potassium ion, and sodium ion, preferably at least one selected from the group consisting of chloride ion, sulfide ion, phosphate ion, and carbonate ion. Examples of the inorganic salt containing chloride ion include sodium chloride, potassium chloride, magnesium chloride, and the like. Examples of the inorganic salt containing sulfide ion include sodium sulfate, potassium sulfate, ammonium sulfate, magnesium sulfate, and the like. Examples of the inorganic salt containing phosphate ion include ammonium phosphate, sodium phosphate, potassium phosphate, and the like. Examples of the inorganic salt containing carbonate ion include sodium carbonate, potassium carbonate, and the like. The inorganic salt is preferably at least one selected from the group consisting of sodium chloride, magnesium chloride, and magnesium sulfate.

### 4. Aqueous dispersion

In the purification method of the present invention, the aqueous dispersion to be subjected to the ultrafiltration step is characterized by containing a virus (described above), a surfactant (described above), and an inorganic salt (described above). The aqueous dispersion may be prepared or obtained by any method as long as it has such characteristics. For example, it may be an aqueous dispersion of virus obtained by extracting the virus from cells containing the virus with an extraction solution containing a surfactant. The inorganic salt may be added simultaneously with the extraction of the virus, or may be added after the extraction. Furthermore, in the purification method of the present invention, the surfactant to be used when extracting the virus and the surfactant to be used when ultrafiltration may be different. In that case, the surfactant is replaced after extracting the virus.

In the present invention, the cell from which the virus-containing cell is derived is not particularly limited as long as the desired virus can be proliferated, and it is preferably a cell into which a part of the gene necessary for virus production has been introduced and is a packaging cell that does not produce virus on its own. It is derived from eukaryotic cells, and preferred examples include HEK293 cell, HEK293T cell, HEK293F cell, HEK293FT cell, G3T-hi cell, Sf9 cell, commercially available cell line for virus production, AAV293 cell, and the like, which have high transfection efficiency. It is preferably HEK293 cell. For example, while the aforementioned HEK293 cell and the like constitutively express the adenovirus E1 protein, the cell may be such cell modified to transiently or constitutively express one or several of the proteins necessary for recombinant adenoviruses.

In one embodiment, examples of the elements necessary for the production of recombinant adenoviruses include (A) AAV-derived Rep protein, Cap protein, (B) adenovirus-derived elements such as E1a, E1b, E2, E4, VARNA gene, and the like. The form of these nucleic acids is not limited, and each element can be provided as one or more nucleic acid constructs, loaded onto a plasmid or viral vector, and introduced into the cells to be used.

Production of the cell containing virus is performed by culturing virus-producing cells obtained by a method including a step of transiently or constitutively introducing a nucleic acid that supplies elements essential for the formation of the virus particles into the cell from which the virus-containing cell is derived. The method for transiently or constitutively introducing the nucleic acid is not particularly limited and, for example, known transient introduction method or constitutive introduction method described above as the introduction method of plasmid may also be used.

The cells can be cultured under known culture conditions. The culture form of cells may be suspension culture or adherent culture. For example, culturing at temperature 30 to 37°C, humidity 95%RH, CO₂ concentration 5 to 10% (v/v) can be mentioned but the culture is not limited thereto. As long as proliferation of virus-producing cells and production of virus particles can be achieved, the culture may be performed at temperature, humidity, and CO₂ concentrations outside the aforementioned ranges. The culture period is not particularly limited, and is, for example, 12 to 150 hr, preferably 48 to 120 hr. The medium used for culturing virus-producing cells may contain components necessary for culturing the cells, and examples thereof include basal synthetic media such as DMEM, IMDM, DMEM:F-12, and the like. These basal synthetic media may be supplemented with fetal bovine serum, growth factors, peptides, or have an increased amount of amino acids, where necessary.

The steps of recovering the virus from virus-containing cells and obtaining an aqueous dispersion can be performed by methods generally practiced in the art. Physical cell-disrupting methods such as mechanical stirring, ultrasonic disrupting, freeze-thawing, and the like, solution extraction method, chemical methods for appropriately adjusting the pH and salt concentration of the solution to be extracted, and a combination of these methods, and the like can be mentioned. For example, in a solution extraction method, cells containing a virus are brought into contact with a surfactant. The method of bringing virus-containing cells into contact with a surfactant is performed by suspending virus-containing cells recovered by centrifugation or filtration in a surfactant, or adding a surfactant to a culture medium containing virus-containing cells. A sufficient amount of the virus can be obtained by adding components necessary for the below-mentioned virus extraction. In the purification method of the present invention, it is necessary to ultrafiltrate the aqueous dispersion containing the virus in the presence of a surfactant and an inorganic salt. Thus, preferably, it is performed by a method using a solution containing a surfactant (described above) as the extraction solution and including adding an inorganic salt (described above) to adjust the salt concentration as appropriate.

The amount of the surfactant to be added during extraction may vary depending on the type of surfactant used, and the final concentration added is generally 0.01 to 2.5%, preferably 0.05 to 2%, more preferably 0.1 to 1.5%, based on the extraction solution. When the amount of the surfactant to be added is too small, a sufficient amount of virus is not obtained, and when it is too large, the virus is inactivated. When two or more types of the surfactants are used in combination, the total amount is adjusted as appropriate so that it falls within the above-mentioned range.

When an inorganic salt is contained during extraction, the amount of the inorganic salt to be added may vary depending on the type of inorganic salt used, and the final concentration added is generally 50 to 1000 mM, preferably 80 to 800 mM, more preferably 100 to 600 mM, based on the extraction solution. When the amount of the inorganic salt to be added is too large or too small, a sufficient amount of virus cannot be achieved. When two or more types of the inorganic salts are used in combination, the total amount is adjusted as appropriate so that it falls within the above-mentioned range.

As used herein, the extraction solution is obtained by adding components necessary for virus extraction other than surfactants and inorganic salts to a cell culture medium or buffer solution, and the components include endonuclease and a small amount of MgCl₂ necessary for the activation thereof. Endonuclease is used to degrade contaminating DNA/RNA, mostly nucleic acids derived from virus-containing cells.

When virus is extracted from virus-containing cells by a method using a solution containing a surfactant (described above) as the extraction solution and including adding an inorganic salt (described above) to adjust the salt concentration as appropriate, incubation is generally performed at 25 to 40°C, preferably 30 to 37°C, for 0.5 to 5 hr, preferably 1 to 3 hr, more preferably about 2 hr.

### 5. Ultrafiltration

In the present invention, an aqueous dispersion containing a virus, a surfactant, and an inorganic salt is subjected to ultrafiltration. The aqueous dispersion to be subjected to the ultrafiltration is not particularly limited as long as it contains a virus and a surfactant and an inorganic salt at predetermined concentrations. It is preferable to use one described in the above-mentioned "4. Aqueous dispersion".

When the aqueous dispersion contains undesired components other than viruses, a step of removing the mixed components, i.e., a clarification step, may be performed before the aqueous dispersion is subjected to ultrafiltration. Clarification can be performed by methods generally practiced in the art, such as filtration through a membrane (filter filtration). The pore size of the membrane is set appropriately depending on the size of the contaminant components to be removed, and is generally within the range of 0.1 to 20 µm, preferably 0.22 to 10 um. As the clarification step, for example, a crude filtration step using a membrane with a large pore size is performed, followed by a precision filtration step using a membrane with a small pore size. Here, the pore size of the membrane used in the crude filtration step is within the range of 1 to 20 um, and the pore size of the membrane used in the precision filtration step is within the range of 0.1 to 1.0 µm.

As the surfactant in the aqueous dispersion to be subjected to ultrafiltration, the same surfactants as those described in the above-mentioned "2. Surfactant" can be used. When a virus is extracted from virus-containing cells by using a surfactant, it is preferable to use the same type of surfactant as the one used at that time. The concentration of the surfactant in the aqueous dispersion is appropriately set according to the type of surfactant to be used, and the final concentration (mass%) is generally set to be 0.01 to 2.0%, preferably 0.03 to 1.5%, more preferably 0.05 to 1.0%, based on the total amount of the aqueous dispersion before being subjected to ultrafiltration. When the amount of the surfactant to be added is too large or too small, a sufficient yield cannot be achieved by ultrafiltration.

As the concentration of the inorganic salt in the aqueous dispersion to be subjected to ultrafiltration, the same concentration as those described in the above-mentioned "3. Inorganic salt" can be used. When the salt concentration is adjusted by adding an inorganic salt during recovery (extraction) of a virus is from virus-containing cells, it is preferable to use the same type of inorganic salt as the one used at that time. The concentration of the inorganic salt in the aqueous dispersion is appropriately set according to the type of inorganic salt to be used, and the final concentration is generally set to be 80 to 800 mM, preferably 100 to 700 mM, more preferably 100 to 600 mM, based on the total amount of the aqueous dispersion before being subjected to ultrafiltration. When the amount of the inorganic salt to be added is too large or too small, a sufficient yield cannot be achieved by ultrafiltration.

When the virus is an adeno-associated virus, the yield in ultrafiltration can be further increased by adjusting the concentration of inorganic salt according to the serotype of AAV.

Ultrafiltration is performed according to methods generally practiced in the art (e.g., Ultrafiltration Handbook, Munir Cheryan (Technomic Publishing, 1986; ISBN No. 87762-456-9)). Preferred filtration step is tangential flow filtration ("TFF"). The tangential flow filtration is also called crossflow filtration, and is a method in which a flow (cross flow or tangential flow) parallel to the membrane surface, which is the filtration direction, is generated by a pump or bubbles, and filtration is performed while removing the deposited layer that forms on the surface of the ultrafiltration membrane. TFF is widely used in the bioprocessing industry for cell recovery, clarification, and purification and concentration of virus-containing products. TFF also includes tangential flow depth filtration (TFDF) which is in combination with a depth filter, single-pass TFF with one filter filtration, and the like. Depth filter is a filter that uses a porous filtration medium to retain particles throughout the medium rather than just on the medium surface. The ultrafiltration membrane used in the present invention has a pore size small enough to retain viruses and large enough to efficiently remove impurities. It is appropriately selected according to the size of the target virus. In the case of a virus belonging to the Retroviridae family, a membrane with a nominal molecular weight cutoff of 100 to 1000 kDa, preferably 100 to 500 kDa, is suitably used. The composition of the membrane is not particularly limited as long as it is porous, and the membrane can be produced from a wide range of polymeric materials known in the art, including, but not limited to, polyolefins such as polyethylene and polypropylene, polyvinylidene fluoride, polyamides, polytetrafluoroethylene, cellulose, polysulfone, polyacrylonitrile, and the like. The membrane can be a flat sheet (also called a flat screen) or a hollow fiber. The preferred ultrafiltration membrane to be used in the present invention is a hollow type fiber ultrafiltration membrane. Ultrafiltration may include, and preferably includes, diafiltration (DF) using an ultrafiltration device.

In the purification method of the present invention, the temperature during ultrafiltration is preferably 2 to 50°C, more preferably 2 to 40°C. It may be 2 to 36°C, 2 to 30°C, or 2 to 26°C. When the temperature is too low, the yield of the virus decreases, and when it is too high, the virus is inactivated.

The temperature during ultrafiltration can be adjusted as appropriate according to the type of target virus and the serotype thereof. For example, the temperature when ultrafiltering an aqueous dispersion containing adeno-associated virus (AAV) is preferably 20 to 50°C, more preferably 22 to 40°C, and further preferably 24 to 36°C.

For example, when using an aqueous dispersion containing an adeno-associated virus (AAV) whose serotype is AAV5 and/or AAV6, the yield of AAV can be increased by adjusting the temperature during ultrafiltration. The temperature during ultrafiltration is, for example, preferably 28 to 50°C, 30 to 45°C, 30 to 40°C, or 32 to 36°C.

The solution obtained by ultrafiltration (ultrafiltration/diafiltration in some cases) can be used as a crude purified liquid (crude liquid) which can be and is preferably subjected to a further purification step.

Further purification step includes purification by column chromatography. Purification techniques by column chromatography are well known to those skilled in the art (e.g., JP-A-2014-237661). One or more (combination) of various chromatography methods can be used for this purification. Examples of the chromatography method include affinity chromatography, ion exchange chromatography, hydrophobic chromatography, gel filtration chromatography, reversed-phase chromatography, immobilized metal ion affinity chromatography, and the like, preferably affinity chromatography, ion exchange chromatography, hydrophobic chromatography, gel filtration chromatography, and combinations thereof (also referred to as multi-mode chromatography, mixed mode chromatography, and the like). The multi-mode chromatography (mixed mode chromatography) is a method utilizing multiple different separation modes (ion exchange, hydrophobic bond, affinity bond, size-exclusion, and the like). Apparatuses, fillers and the like used for these chromatography methods are also commercially available.

The present invention is described in detail below by using Examples, but the present invention is not limited in any way. Unless otherwise specified, the reagents and materials used are commercially available or can be prepared from known literatures. In addition, those skilled in the art understand that they can be substituted with those having similar effects and actions.

### [Example]

### Example 1

### 1. Production of viral vector

AAV2 vector expressing GFP was produced by triple transfection of HEK293 cells. The specific steps are shown below.

HEK293 cells were cultured in a hyper flask using DMEM supplemented with 10% FBS. Thereafter, the culture was continued in a CO₂ incubator at 37°C, and it was confirmed that the cells reached about 70% confluent.

After replacing the cell culture medium with fresh DMEM (containing 10% FBS), the following plasmids (i) to (iii) were used for transfection.
(i) Plasmid encoding AAV2 Rep protein and Cap protein (Takara Bio Inc., pRC2-mi342 Vector)
(ii) Plasmid containing adenovirus E2A sequence, VA sequence, and E4 sequence (Takara Bio Inc., pHelper Vector)
(iii) Plasmid containing an expression cassette for the fluorescent protein GFP between the two ITRs of AAV2 (CELL BIOLABS, pAAV-GFP)

### 2. Viral vector extraction

Three to five days after transfection, the non-ionic surfactant Tween 20 (final concentration 0.5%), Endonuclease (final concentration 50 U/mL), and MgCl₂ (final concentration 2-3 mM) were added to an HEK293 cell culture medium and the cells were incubated (37°C) for 2 hr. At the same time as extraction of the AAV2 vector using Tween 20, nucleic acid treatment is performed using Endonuclease. MgCl₂ is required for Endonuclease activity. After recovery, the flask was washed with 200 mL of D-PBS and mixed with the content.

Next, NaCl was added to a final concentration of 390 mM, and after incubation for 1 hr (37°C), the AAV vector-containing material was recovered. The mixture was filtered through a 0.45 um filter, and the filtrate containing the AAV vector was recovered as a clarified vector solution (virus-containing aqueous dispersion). The concentration of inorganic salt (NaCl) in the virus-containing aqueous dispersion after recovery was 390 mM, and the concentration of the surfactant was 0.35% by mass.

### 3. Virus purification

Ultrafiltration was performed to concentrate the virus-containing aqueous dispersion. The temperature during ultrafiltration was 25°C. In order to perform buffer exchange simultaneously with the ultrafiltration, ultrafiltration/diafiltration (UF/DF) using tangential flow filtration (TFF) was performed. Ultrafiltration and diafiltration were performed by concentrating the sample about 10 times using AKTA flux s (Cytiva) with a 300 kDa hollow fiber membrane whose membrane material is polysulfone, after which buffer was exchanged using D-PBS in an amount 6.3 times that of the concentrate. The titer of the AAV vector was determined by qPCR from the amount of viral genome. The yield after ultrafiltration is shown in Table 1.

### Example 2

Ultrafiltration was performed in the same manner as in Example 1 except that the concentration of surfactant in the virus-containing aqueous dispersion after recovery in "2. Viral vector extraction" in Example 1 was set to 0.07% by mass. The results are shown in Table 1.

### Example 3

Ultrafiltration was performed in the same manner as in Example 1 except that the concentration of surfactant in the virus-containing aqueous dispersion after recovery in "2. Viral vector extraction" in Example 1 was set to 0.50% by mass. The results are shown in Table 1.

### Example 4

Ultrafiltration was performed in the same manner as in Example 1 except that the concentration of surfactant in the virus-containing aqueous dispersion after recovery in "2. Viral vector extraction" in Example 1 was set to 1.0% by mass. The results are shown in Table 1.

### Example 5

Ultrafiltration was performed in the same manner as in Example 1 except that the concentration of inorganic salt (NaCl) in the virus-containing aqueous dispersion after recovery in "2. Viral vector extraction" in Example 1 was set to 210 mM. The results are shown in Table 1.

### Example 6

Ultrafiltration was performed in the same manner as in Example 1 except that the concentration of inorganic salt (NaCl) in the virus-containing aqueous dispersion after recovery in "2. Viral vector extraction" in Example 1 was set to 410 mM. The results are shown in Table 1.

### Example 7

Ultrafiltration was performed in the same manner as in Example 1 except that the concentration of inorganic salt (NaCl) in the virus-containing aqueous dispersion after recovery in "2. Viral vector extraction" in Example 1 was set to 600 mM. The results are shown in Table 1.

### Example 8

The inorganic salt to be added in "2. Viral vector extraction" in Example 1 was changed from NaCl to MgSO₄ and inorganic salt (MgSO₄) was added such that the concentration in the virus-containing aqueous dispersion after recovery was 110 mM. Furthermore, since the activity of Endonuclease decreases in the presence of NaCl, NaCl was added after 2 hr of incubation in Example 1, rather than at the same time as Tween20, Endonuclease, and MgCl₂. In Example 8, since MgSO₄ does not inhibit endonuclease activity, MgSO₄ was added simultaneously with Tween20, Endonuclease, and MgCl₂. The results obtained using the same method as in Example 1 for the rest are shown in Table 1.

### Example 9

The inorganic salt to be added in "2. Viral vector extraction" in Example 8 was changed from MgSO₄ to MgClz and inorganic salt (MgCl₂) was added such that the concentration in the virus-containing aqueous dispersion after recovery was 100 mM. The results obtained using the same method as in Example 8 for the rest are shown in Table 1.

### Example 10

Ultrafiltration was performed in the same manner as in Example 1 except that the surfactant in the virus-containing aqueous dispersion after recovery in "2. Viral vector extraction" in Example 1 was Tween80. The results are shown in Table 1.

### Example 11

Ultrafiltration was performed in the same manner as in Example 1 except that the surfactant in the virus-containing aqueous dispersion after recovery in "2. Viral vector extraction" in Example 1 was Pluronic (registered trade mark) F-68. The results are shown in Table 1.

### Example 12

The serotype of the viral vector in "1. Production of viral vector" in Example 1 was changed to AAV1. The specific steps are shown below.

HEK293 cells were cultured in a hyper flask using DMEM supplemented with 10% FBS. Thereafter, the culture was continued in a CO₂ incubator at 37°C, and it was confirmed that the cells reached about 70% confluent.

After replacing the cell culture medium with fresh DMEM (containing 10% FBS), the following plasmids (i) to (iii) were used for transfection.
(i) Plasmid encoding AAV1 Rep protein and Cap protein (Takara Bio Inc., pRC1 Vector)
(ii) Plasmid containing adenovirus E2A sequence, VA sequence, and E4 sequence (Takara Bio Inc., pHelper Vector)
(iii) Plasmid containing an expression cassette for the fluorescent protein GFP between the two ITRs of AAV2 (CELL

### BIOLABS, pAAV-GFP)

The rest was performed by a method similar to that in Example 1 and the results are shown in Table 1.

### Example 13

The serotype of the viral vector in "1. Production of viral vector" in Example 1 was changed to AAV5. The specific steps are shown below.

HEK293 cells were cultured in a hyper flask using DMEM supplemented with 10% FBS. Thereafter, the culture was continued in a CO₂ incubator at 37°C, and it was confirmed that the cells reached about 70% confluent.

After replacing the cell culture medium with fresh DMEM (containing 10% FBS), the following plasmids (i) to (iii) were used for transfection.
(i) Plasmid encoding AAV5 Rep protein and Cap protein (Takara Bio Inc., pRC5 Vector)
(ii) Plasmid containing adenovirus E2A sequence, VA sequence, and E4 sequence (Takara Bio Inc., pHelper Vector)
(iii) Plasmid containing an expression cassette for the fluorescent protein GFP between the two ITRs of AAV2 (CELL

### BIOLABS, pAAV-GFP)

The rest was performed by a method similar to that in Example 1 and the results are shown in Table 1.

### Example 14

The serotype of the viral vector in "1. Production of viral vector" in Example 1 was changed to AAV6. The specific steps are shown below.

HEK293 cells were cultured in a hyper flask using DMEM supplemented with 10% FBS. Thereafter, the culture was continued in a CO₂ incubator at 37°C, and it was confirmed that the cells reached about 70% confluent.

After replacing the cell culture medium with fresh DMEM (containing 10% FBS), the following plasmids (i) to (iii) were used for transfection.
(i) Plasmid encoding AAV6 Rep protein and Cap protein (Takara Bio Inc., pRC6 Vector)
(ii) Plasmid containing adenovirus E2A sequence, VA sequence, and E4 sequence (Takara Bio Inc., pHelper Vector)
(iii) Plasmid containing an expression cassette for the fluorescent protein GFP between the two ITRs of AAV2 (CELL

### BIOLABS, pAAV-GFP)

The rest was performed by a method similar to that in Example 1 and the results are shown in Table 1.

### Example 15

Ultrafiltration was performed in the same manner as in Example 1 except that the temperature in the sample tank was set to 32 to 36°C during ultrafiltration in Example 13, and the results are shown in Table 1.

### Example 16

Ultrafiltration was performed in the same manner as in Example 14 except that the temperature in the sample tank was set to 32 to 36°C during ultrafiltration in Example 14, and the results are shown in Table 1.

**[Table 1]**

| Example | serotype | inorganic salt | | surfactant | | temperature (°C) | recovery rate (%) |
|---|---|---|---|---|---|---|---|
| | | kind | concentration (mM) | kind | concentration (mass %) | | |
| Ex. 1 | AAV2 | NaCl | 390 | Tween20 | 0.35 | 25 | 100 |
| Ex. 2 | AAV2 | NaCl | 390 | Tween20 | 0.07 | 25 | 81 |
| Ex. 3 | AAV2 | NaCl | 390 | Tween20 | 0.50 | 25 | 90 |
| Ex. 4 | AAV2 | NaCl | 390 | Tween20 | 1.0 | 25 | 87 |
| Ex. 5 | AAV2 | NaCl | 210 | Tween20 | 0.35 | 25 | 79 |
| Ex. 6 | AAV2 | NaCl | 410 | Tween20 | 0.35 | 25 | 94 |
| Ex. 7 | AAV2 | NaCl | 600 | Tween20 | 0.35 | 25 | 106 |
| Ex. 8 | AAV2 | MgSO₄ | 110 | Tween20 | 0.35 | 25 | 100 |
| Ex. 9 | AAV2 | MgCl₂ | 100 | Tween20 | 0.35 | 25 | 83 |
| Ex. 10 | AAV2 | NaCl | 390 | Tween80 | 0.35 | 25 | 99 |
| Ex. 11 | AAV2 | NaCl | 390 | Pluronic F-68 | 0.35 | 25 | 100 |
| Ex. 12 | AAV1 | NaCl | 390 | Tween20 | 0.35 | 25 | 88 |
| Ex. 13 | AAV5 | NaCl | 390 | Tween20 | 0.35 | 25 | 69 |
| Ex. 14 | AAV6 | NaCl | 390 | Tween20 | 0.35 | 25 | 83 |
| Ex. 15 | AAV5 | NaCl | 390 | Tween20 | 0.35 | 32-36 | 89 |
| Ex. 16 | AAV6 | NaCl | 390 | Tween20 | 0.35 | 32-36 | 92 |

In Table 1, the concentration means the concentration in the virus-containing aqueous dispersion.

From the results in Table 1, it is considered that adsorption of viral vectors to membranes during ultrafiltration can be prevented by adding a surfactant and an inorganic salt at certain concentrations to the viral vector extract (virus-containing aqueous dispersion), and the yield is improved.
This is considered to be because the inorganic salt suppresses aggregation of the viral vector.

In addition, the yield can be improved by adjusting the temperature during ultrafiltration.

### [Industrial Applicability]

According to the method of the present invention, it becomes possible to purify viruses more conveniently at a low cost in a high yield.

This application is based on a patent application No. 2021-184941 filed in Japan (filing date: November 12, 2021), the contents of which are incorporated in full herein.

## Claims

1. A method for purifying a virus, comprising a step of ultrafiltrating an aqueous dispersion comprising a virus, a surfactant, and an inorganic salt.

2. The purification method according to claim 1, wherein the inorganic salt is an inorganic salt containing at least one selected from the group consisting of chloride ion, sulfide ion, phosphate ion, and carbonate ion.

3. The purification method according to claim 1, wherein the inorganic salt is at least one selected from the group consisting of sodium chloride, magnesium chloride, and magnesium sulfate.

4. The purification method according to claim 1, wherein the surfactant is at least one selected from the group consisting of a non-ionic surfactant, an anionic surfactant, a cationic surfactant, and a zwitterionic surfactant.

5. The purification method according to claim 1, wherein a content of the surfactant is 0.01 to 2.0 mass % with respect to the total amount of the aqueous dispersion.

6. The purification method according to claim 1, wherein a content of the inorganic salt is 80 to 800 mM with respect to the total amount of the aqueous dispersion.

7. The purification method according to claim 1, further comprising a step of extracting a virus from a cell containing the virus and obtaining the aqueous dispersion, which step is performed before the ultrafiltration step.

8. The purification method according to claim 1, further comprising a step of purifying by column chromatography, which step is performed after the ultrafiltration step.

9. A method for purifying a virus, comprising
(a) a step of extracting a virus from a cell containing the virus and obtaining an aqueous dispersion containing the virus, a surfactant, and an inorganic salt,
(b) a step of performing ultrafiltration of the aqueous dispersion to obtain a crude liquid containing the virus, and
(c) a step of purifying the crude liquid by column chromatography.
